# EUROPÄISCHE PATENTSCHRIFT

(11) **EP 3 585 326 B1**
(45) Veröffentlichungstag und Bekanntmachung des Hinweises auf die Patenterteilung: **14.04.2021**
(21) Anmeldenummer: 18706700.4
(22) Anmeldetag: 16.02.2018
(51) Int. Cl.: A61F 5/56

(54) **UNTERKIEFER-PROTRUSIONSSCHIENE**
MANDIBLE PROTRUSION RAIL
GOUTTIÈRE DE PROPULSION MANDIBULAIRE

(30) Priorität: 23.02.2017 DE 102017103722
(43) Veröffentlichungstag der Anmeldung: 01.01.2020
(73) Patentinhaber: Hicat GmbH, 53177 Bonn (DE)
(72) Erfinder: HEY, Joachim, 53639 Königswinter (DE); GRÜNBERG, Daniel, 53179 Bonn (DE); FREYER, Dirk, 50937 Köln (DE)
(74) Vertreter: Braun-Dullaeus Pannen Emmerling Patent- & Rechtsanwaltspartnerschaft mbB
(86) Internationale Anmeldenummer: PCT/EP2018/053924
(87) Internationale Veröffentlichungsnummer: WO 2018/153789

(56) Entgegenhaltungen:
- EP-A1- 3 103 420
- WO-A1-92/19174
- WO-A1-2015/132406
- DE-A1-102010 046 369
- US-A1- 2015 216 716

## Beschreibung

Die Erfindung betrifft eine Unterkiefer-Protrusionsschiene zur Positionierung des Unterkiefers gegenüber dem Oberkiefer eines Patienten aufweisend eine Oberkieferteilschiene und eine Unterkieferteilschiene, wobei zwei seitlich an den Teilschienen angebrachte Gelenkstangen die beiden Teilschienen beweglich miteinander koppeln. Zur Halterung der Gelenkstangen weisen die beiden Teilschienen seitlich, respektive lateral oder temporal, angebrachte Gelenkbolzen auf, die eine der Teilschiene zugewandte Schulter, einen der Teilschiene abgewandten Kopf und einen zwischen Schulter und Kopf befindlichen Hals mit geringerem Durchmesser haben. Die Gelenkstangen haben an ihren Enden jeweils einen Kragen, der im montierten Zustand den Hals eines Gelenkbolzens umgibt, wobei die Gelenkstange über den Kragen zwischen der Schulter und dem Kopf, die jeweils einen größeren Querschnitt als der Hals haben, gehalten ist.

Eine derartige Protrusionsschiene ist beispielsweise aus dem DE 20 1008 011 841 U1 bekannt. Auch die EP 3 103 420 A1 und die DE 10 2010 46 369 A1 offenbaren gattungsgemäße Protrusionsschienen.

Solche Unterkiefer-Protrusionsschienen werden zur Therapie von obstruktiver Schlafapnoe (OSA) eingesetzt. Sie verhindern durch eine entsprechende Positionierung des Unterkiefers eine durch OSA hervorgerufenen pathologische Einschränkung der Atemfunktion während des Schlafs. Solche zweiteiligen Unterkiefer-Protrusionsschienen mit einstellbarer Therapieposition - respektive Protrusion - sind seit längerem bekannt. Dabei wird die mechanische Kopplung zwischen der Oberkieferteilschiene und der Unterkieferteilschiene unter Einhaltung der gewünschten Therapieposition auf unterschiedliche Weise umgesetzt.

Die beiden Teilschienen sind über Verbindungselemente, die als Gelenkstangen ausgebildet sind, miteinander verbunden, wobei die Geometrie und die Aufhängung der Gelenkstangen den Grad der Protrusion bestimmen und den Raum der möglichen Bewegungen des Unterkiefers maßgeblich beeinflussen. Dabei lassen die bekannten Unterkiefer-Protrusionsschienen auch der seitlichen Bewegung des Unterkiefers einen gewissen Bewegungsraum, der jedoch nur einem Teilbereich des anatomisch möglichen Bewegungsraums des Unterkiefers entspricht. Dadurch, dass der tatsächliche Bewegungsraum des Unterkiefers größer als der von der Unterkiefer-Protrusionsschiene zugelassene Bewegungsraum ist, kann es an den Grenzbereichen des durch die Protrusionsschiene vorgegebenen Bewegungsraums zu starken mechanischen Belastungen der Teilschienen und des Patienten kommen. Diese Belastungen können die Schiene beschädigen und im schlimmsten Fall dem Patienten Verletzungen zufügen.

WO 2015/132406 A1 stellt den nächstliegenden Stand der Technik dar und beschreibt eine entfernbare Vorrichtung zum Vorschieben eines Unterkiefers gemäß des Oberbegriffs von Anspruch 1.

Aufgabe der vorliegenden Erfindung ist es nunmehr, eine Unterkiefer-Protrusionsschiene zu schaffen, die bei einfachem Aufbau und bei hohem Tragekomfort ein großes Maß an Bruchsicherheit bietet und damit die Gefahr von Verletzungen erheblich reduziert.

Diese Aufgabe wird durch eine Unterkiefer-Protrusionsschiene mit den Merkmalen des Anspruch 1 gelöst. Vorteilhafte Ausführungsformen sind in den Unteransprüchen genannt.

Entsprechend dem Anspruch 1 liegt der Kern der Erfindung in einer konstruktiv bedingten Flexibilität am Kragen der Gelenkstange und/oder einer konstruktiv bedingten Flexibilität am Kopf des Gelenkbolzens, die bei übermäßiger seitlicher Kraftbeaufschlagung zu einem Nachgeben eines der Bauteile führt, so dass die Gelenkstange im Extremfall mit dem Kragen zerstörungsfrei über den Kopf des Gelenkbolzens ausrücken kann. Diese Funktion wird insbesondere von einer besonderen Formgebung des Kopfes des Gelenkbolzens und/oder von dessen Materialwahl unterstützt. Als übermäßige seitliche Kraftbeaufschlagung werden insbesondere solche Kräfte verstanden, die herkömmlichen Protrusionsschienen zur Zerstörung führen würden und die von Bewegungen verursacht werden, die von überdehnten Bewegungen am Rande des anatomisch Möglichen liegen. Mit der Erfindung kann ein quasi zweistufiges Nachgeben realisiert werden, wobei die Gelenkstange entweder so über den Gelenkbolzen geführt wird, dass sie sich bei seitlicher Kraftbeaufschlagung in einer reversiblen Ausweichbewegung zunächst aus der Bewegungsbahn der anderen Teilschiene herausbewegt. Bei weiterer Kraftbeaufschlagung über das Ende der reversiblen Ausweichbewegung hinaus, rückt die Gelenkstange dann zerstörungsfrei über den Kopf des Gelenkbolzens aus, indem sich der Kragen etwas weitet und über den Kopf des Gelenkbolzens rutscht und/oder indem ein Teil des Kopfes insbesondere im Bereich des Hinterkopfes material- oder formbebedingt nachgibt.

Die Abstufungen sind auch durch die individuelle Anpassung der Protrusionsschiene an die Anatomie des Patienten bedingt. Dabei bewegen sich die Teilschienen idealerweise auf einer horizontalen Fläche, die über die Berührungsflächen der Teilschienen definiert ist. Anatomisch bedingt, können die Teilschienen bei extremen Bewegungen aus der Ebene heraus kippen. Wenn es zu einer solchen Verkippung kommt, kann die Überlastung zuletzt nicht mehr durch eine Ausweichbewegung der Gelenkstange verhindert werden, da sich diese trotz Ausweichbewegung noch im Bewegungsraum der anderen Teilschiene befindet. In solchen Extremfällen wird die Überlast durch das Ausrücken der Gelenkstange abgebaut und die Zerstörung der Protrusionsschiene vermieden.

In der besonders zu bevorzugenden Ausführungsform der erfindungsgemäßen Unterkiefer-Protrusionsschiene wird die mechanische Kopplung über zwei als Verbindungselemente wirkende Gelenkstangen, jeweils eine pro Seite, umgesetzt. Jeder Gelenkstange ist jeweils ein Aufnahmeelement in Form eines insbesondere angeformten Gelenkbolzens an der Ober- und der Unterkieferteilschiene zugeordnet.

Die Gelenkbolzen halten die Gelenkstangen beweglich in ihrer Position. Sie sind in drei Teilbereiche, nämlich den Kopf, den Hals und die Schulter, unterteilt. Die Schulter und der Hals sind bevorzugt rotationssymmetrisch bezüglich der Achse des Gelenkbolzens ausgebildet, wobei die Schulter den Übergang des Halses zur Teilschiene bildet. Der Hals ist der zentrale Teil des Gelenkbolzens, der die eigentliche Aufnahme für die Gelenkstange bildet, wobei die montierte Gelenkstange den Hals mit einem am Ende ausgebildeten Kragen umfasst. An den Hals des Gelenkbolzens schließt sich nach außen hin der Kopf an, der bei normaler Verwendung der Protrusionsschiene ein Abrutschen der Gelenkstange vom Gelenkbolzen verhindert. Die Gelenkstange hat dazu an jedem Ende einen als Aussparung ausgebildeten Kragen, den der Gelenkbolzen im montierten Zustand durchragt. Vorteilhafterweise bildet der Kragen eine als Langloch geformte Ausnehmung. Der Durchmesser der Aussparung des Kragens ist so bemessen, dass er im Ausgangszustand kleiner als der Durchmesser des Gelenkbolzenkopfes ist, was ein Ausrücken ohne Dehnung des Kragens der Gelenkstange verhindert.

Die erfindungsgemäße Flexibilität ergibt sich vorteilhafterweise aus dem Material respektive der Materialpaarung, aus der die Teile der Protrusionsschiene gefertigt sind. Die Flexibilität wird durch die Formung des Gelenkbolzens insofern unterstützt, als Gleit- und Anlageflächen geschaffen sind, die zunächst ein Kippen der Gelenkstange und dann ein langsames Aufweiten des Kragens hervorrufen. Dabei sind in einer besonders bevorzugten Ausführungsform die beiden Teilschienen mit den daran angeformten Gelenkbolzen aus einem gegenüber der Gelenkstange härteren Material, insbesondere aus Kunststoff, beispielsweise aus PMMA, gefertigt. Die Teilschienen sind idealerweise patientenindividuell gefertigt und liegen unverschieblich auf den Zähnen der Kiefer auf. Die beiden seitlich vermittels der Gelenkbolzen angebrachten Gelenkstangen sind bevorzugt aus einem weniger harten Material als die Teilschienen, vorteilhafterweise auch aus einem Kunststoff, beispielsweise einem Polyamid, gefertigt. Somit liegt die beanspruchte Flexibilität in der Gelenkstange, deren Kragen sich bei einer übermäßigen Belastung um ein gewisses Maß weitet, so dass der Gelenkbolzen ausrücken kann. Zusätzlich sorgt die Form der Gelenkbolzen dafür, dass die Gelenkstange die Ausweichbewegung entlang des Gelenkbolzens ausführen kann, dadurch dass die abgerundete Form im Übergang von Hals zu Kopf die Gelenkstange führt und nicht blockiert.

Dabei kommt es zu einer übermäßigen Belastung, wenn der Patient mit einer Kieferbewegung insbesondere die lateralen Grenzen des durch die Unterkiefer-Protrusionsschiene vorgegebenen Bewegungsraums überschreitet. Während es bei den bekannten Protrusionsschiene in einer solchen Situation zum Bruch entweder des Gelenkbolzens oder der Gelenkstange kam, führt eine solche Überlastung bei der erfindungsgemäßen Protrusionsschiene lediglich zu einer Ausweichbewegung der Gelenkstange aus dem Bewegungsraum oder zu einer reversiblen Entkopplung von Gelenkbolzen und Gelenkstange.

Somit kann durch die erfindungsgemäße Ausgestaltung respektive durch die spezielle Geometrie der Teilschienen mit ihren Gelenkbolzen und der als Gelenkstangen ausgebildeten Verbindungselemente, mit denen die beiden Teilschienen verbunden sind, eine Überbelastung reversibel gedämpft und die Protrusionsschiene im Überlastfall durch den erfindungsgemäßen Schutzmechanismus vor Zerstörung und der Patient damit vor Verletzung bewahrt werden.

In einer besonderen Ausführungsform ist der Kopf des Gelenkbolzens nicht rotationsymmetrisch geformt. Idealerweise hat der asymmetrische Kopf des Gelenkbolzens einen erhabenen Vorderkopf und einen flachen Hinterkopf, so dass eine gewisse Hakenform entsteht. Der flache Hinterkopf ist dabei der jeweils anderen Teilschiene zugewandt. Die Vorteile der erfindungsgemäßen Geometrie des Gelenkbolzens liegen in dem daraus resultierenden Verhalten des gesamten Verbindungsmechanismus der Protrusionsschiene bei seitlicher Belastung durch eine vom Patienten ausgeführte Seitwärtsbewegung des Unterkiefers. Erfindungsgemäß kann die Belastung der Patientenanatomie, die beispielsweise zu einer Zahnlockerung oder einer Schädigung der Kiefergelenke und/oder der Kaumuskulatur führt, vermieden werden. Zudem kann ein Defekt der Protrusionsschiene hervorgerufen durch eine Kollision der Teilschienen mit dem Verbindungselement durch eine Ausweichbewegung des Verbindungselements reduziert und so eine Überbelastung verhindert werden.

Die erfindungsgemäße Unterkiefer-Protrusionsschiene wird nachfolgend anhand der
Figuren 1 bis 4 näher beschrieben. Es zeigen:
   - **Figur 1**: eine Unterkiefer-Protrusionsschiene im Mund eines Patienten,
   - **Figur 2**: eine Gelenkstange einer Unterkiefer-Protrusionsschiene,
   - **Figur 3**: einen Schnitt durch den Kopf eines Gelenkbolzens,
   - **Figur 4a**: einen Schnitt in Tragesituation im Normalzustand,
   - **Figur 4b**: einen Schnitt in Tragesituation belasteten Zustand nach einer Ausweichbewegung und
   - **Figur 4c**: einen Schnitt in Tragesituation ausgerückten Zustand.

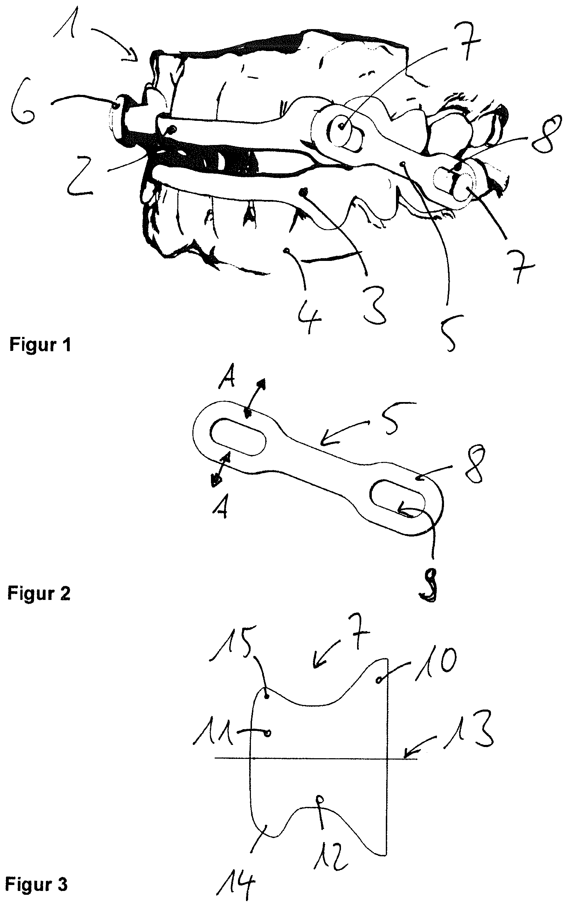

Figur 1 zeigt eine Unterkiefer-Protrusionsschiene, die zwischen dem bezahnten Oberkiefer 1 und dem bezahnten Unterkiefer 4 eines Patienten getragen wird. Sowohl die Oberkieferteilschiene 2 als auch die Unterkieferteilschiene 3 sind individuell an die Bezahnung des Patienten angeformt. Die Oberkieferteilschiene 2 ist mit der Unterkieferteilschiene 3 nur über zwei seitlich angebrachte Gelenkstangen 5 und 6 (hier ist nur das Ende zu sehen) beweglich miteinander verbunden. Über die Länge der Gelenkstangen 5 und 6 wird insofern die Protrusion des Unterkiefers eingestellt, als der Unterkiefer durch die Gelenkstangen 5 und 6 gehindert ist, nach hinten bewegt zu werden. Zur Halterung der Gelenkstangen 5 und 6 weisen die beiden Teilschienen seitlich, respektive lateral oder temporal, angeformte Gelenkbolzen 7 auf, die in den Gelenkstangen 5 vorgesehene Kragen 8 in Form von Langlöchern durchdringen. Durch die Langlöcher ist eine Beweglichkeit der beiden Teilschienen 2 und 3 insbesondere in sagittaler Richtung gegeben.
Figur 2 zeigt eine einzelne Gelenkstange 5 mit einem massiven Mittelteil und mit den an den Enden vorgesehenen Kragen 8, die als Langlöcher ausgeformt sind. Die Gelenkstange 5 ist einteilig aus einem Polyamid gespritzt und dabei derart dimensioniert, dass die Kragen 8 eine gewisse Flexibilität in Richtung der Pfeile A aufweisen. Die Öffnung des Langlochs kann sich entsprechend beim Überstreifen des Kragens über den flachen Hinterkopf des Gelenkbozens reversibel weiten.

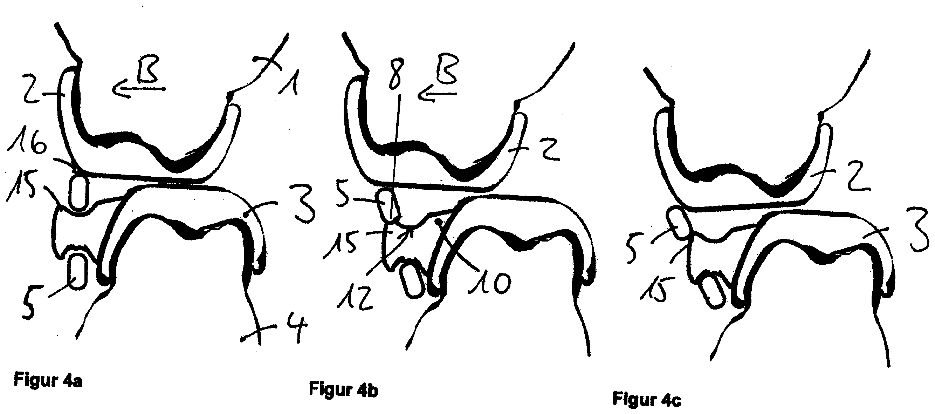

Figur 3 zeigt einen Schnitt durch einen Gelenkbolzen 7, der über eine Schulter 10 an der nicht dargestellten Teilschiene angeformt ist. Der Teilschiene abgewandt hat der Gelenkbolzen einen Kopf 11, so dass sich zwischen Schulter 10 und Kopf 11 ein Hals 12 ausbildet, der in Bezug auf die Achse 13 einen geringeren Durchmesser als Schulter 10 und Hals 11 aufweist. Ersichtlich ist der Kopf des Gelenkbolzens asymmetrisch und bildet einen vorstehenden Vorderkopf 14 und einen flachen Hinterkopf 15 auf. Wie sich aus den Figuren 4 ersehen lässt, ist der flache Hinterkopf 15 der jeweils anderen Teilschiene - hier der Oberkieferteilschiene - zugewandt. Wie sich aus den Figuren 3 und 4 ebenfalls ergibt, sind die sich berührenden Flächen des

Gelenkbolzens und des Kragens der Gelenkstange als kantenfreie Gleitflächen geformt.

Im montierten Zustand umgibt der Kragen 8 den Hals 12 eines Gelenkbolzens, wobei die Gelenkstange 5 zwischen der Schulter 10 und dem Kopf 11, die jeweils einen größeren Querschnitt als der Hals 12 haben, gehalten ist.

Die Wirkungsweise der erfindungsgemäßen Lagerung der Gelenkstange auf den Gelenkbolzen lässt sich wie folgt anhand der Figuren 4 beschreiben:
Da die Unterkiefer-Protrusionsschiene ein patientenindividuelles Design aufweist, ist die Lage der Gelenkbolzen auf den entsprechenden Teilschienen von Patient zu Patient unterschiedlich. Je nach Situation kann es notwendig werden, den Gelenkbolzen in unterschiedlichen Höhen am Außenrand der Teilschienen zu positionieren.

In Figur 4a ist die Situation gezeigt, in welcher der Patient seinen Oberkiefer 1 übermäßig seitlich in Richtung des Pfeiles B bewegt und die gegenüberliegende Teilschiene 2 in die Trajektorie der Gelenkstange 5 hineinreicht, so dass die Teilschiene 2 mit der Gelenkstange 5 an dem Punkt 16 kollidiert.

Bewegt sich der Oberkiefer wie in Figur 4b trotz der Kollision weiter in diese Richtung (Pfeil B), so führt die besondere Gestaltung des Übergangs von der Schulter 10 über den Hals 12 zu dem asymmetrischen Kopf 7 dazu, dass sich die Gelenkstange 5 verkippt und die eine Seite des Kragens 8 zunächst auf der Seite des Vorderkopfes 14 an die Schulter 10 anlegt, während die andere Seite des Kragens 8 gegen den Hinterkopf 15 stößt. Damit bewegt sich die Gelenkstange aus der Bewegungsrichtung der anderen Teilschiene heraus, so dass eine Überlast vermieden wird. Bewegt sich die andere Teilschiene wieder zurück (entgegen Pfeil B) richtet sich die Gelenkstange auf; die Funktion der Protrusionsschiene ist nicht weiter beeinträchtigt.

Dabei wird die Neigungsbewegung durch den asymmetrisch gestalten Kopf, insbesondere durch die Abflachung des Übergangs vom Hals zu Kopf, realisiert, wobei sich die Abflachung, mithin der Hinterkopf, bei den Gelenkbolzen jeweils an der der Gegenschiene zugewandten Seite befindet. Im Vergleich zum Mittelpunkt des Halses ist der abgeflachte Hinterkopf jedoch erhöht, so dass der Abstand der äußeren Kante des Hinterkopfes zur Achse der gegenüberliegenden Teilschiene größer als der Abstand des Radius des Halses ist. Diese Erhöhung besitzt folglich einen größeren Querschnitt als der Kragen, so dass es bei einer fortgesetzten Neigungsbewegung der Gelenkstange zu einer reversiblen mechanischen Dehnung des Kragens kommt, die einen Dämpfungseffekt der Krafteinwirkung des Gegenkiefers zur Folge hat.

Wenn die zum Beispiel durch eine Verkippung aus der Horizontalebene der Protrusionsebene hervorgerufene Belastung durch die gegenüberliegende Teilschiene die mechanische Verformung wie in Figur 4c derart überschreitet, dass sie höher ist, als die durch die plastische Verformung der Gelenkstange hervorgerufene Kraft am höchsten Punkt des Hinterkopfes 15, löst rückt die Gelenkstange 5 über den Kopf 7 des Gelenkbolzens aus, so dass eine Beschädigung der Teilschiene oder eine Verletzung des Patienten verhindert wird. Damit ist die Oberkieferteilschiene 2 zumindest auf diese Seite entkoppelt von der Unterkieferteilschiene 3.

Die beiden Aspekte, nämlich die mögliche Ausweichbewegung der mit einem flexiblen Kragen ausgestatteten Gelenkstange aus der Trajektorie der Gegenschiene einschließlich der unbeschadeten Zurückbewegung der Gelenkstange in die ursprüngliche Ausrichtung sowie das Abheben der Gelenkstange von dem Gelenkbolzen bei Überlast ohne die Protrusionsschiene zu beschädigen oder den Patienten zu verletzen unterscheidet die hier erfindungsgemäße Lösung von den bekannten Unterkiefer-Protrusionsschienen.

## Patentansprüche

1. Unterkiefer-Protrusionsschiene zur Positionierung des Unterkiefers (4) gegenüber dem Oberkiefer (1) eines Patienten aufweisend eine
Oberkieferteilschiene (2) und eine Unterkieferteilschiene (3), wobei Gelenkstangen (5) die beiden Teilschienen (2,3) beweglich miteinander koppeln, wobei die beiden Teilschienen (2,3) seitlich angebrachte Gelenkbolzen (7) zur Halterung der Gelenkstangen (5) aufweisen, wobei die Gelenkbolzen (7) eine der Teilschiene zugewandte Schulter (10), einen der Teilschiene abgewandten Kopf (11) und einen zwischen Schulter (10) und Kopf (11) befindlichen Hals (12) aufweisen, wobei die Gelenkstangen an ihren Enden jeweils einen Kragen (8) aufweisen, der im montierten Zustand der Gelenkstange (5) den Hals (12) eines Gelenkbolzens (7) umgibt, wobei die Gelenkstange (5) zwischen der Schulter (10) und dem Kopf (11), die jeweils einen größeren Querschnitt als der Hals (12) haben, gehalten ist, wobei eine konstruktiv vorgesehene Flexibilität am Kragen (8) der Gelenkstange (5) und/oder am Kopf (11) des Gelenkbolzens (7) bei übermäßiger Beaufschlagung mit einer über die Gelenkstange (5) ausgeübten seitwärts gerichteten Kraft zunächst eine Ausweichbewegung der Gelenkstange (5) ermöglicht,
**dadurch gekennzeichnet, dass** die konstruktiv vorgesehene Flexibilität bei Überlast dann ein zerstörungsfreies Überstreifen des Kragens (8) über den Kopf (11) des Gelenkbolzens (7) ermöglicht.

2. Protrusionsschiene nach Anspruch 1, **dadurch gekennzeichnet, dass** die Ausweichbewegung der Gelenkstange (5) in Richtung des Kopfes (11) erfolgt.

3. Protrusionsschiene nach Anspruch 1 oder 2,
**dadurch gekennzeichnet, dass**
die beiden Teilschienen (2,3) mit angeformten Gelenkbolzen (7) aus einem härteren Kunststoff, insbesondere aus PMMA, als die Gelenkstange (5) gefertigt ist, wobei die Gelenkstange insbesondere im Spritzgussverfahren aus einem Polyamid hergestellt ist.

4. Protrusionsschiene nach einem der vorherigen Ansprüche,
**dadurch gekennzeichnet, dass**
der Kopf (11) eines Gelenkbolzens (7) asymmetrisch mit einem erhabenen Vorderkopf (14) und einen flachen Hinterkopf (15) ausgebildet ist, wobei der flache Hinterkopf (15) der anderen Teilschiene zugewandt ist.

5. Protrusionsschiene nach einem der vorherigen Ansprüche,
**dadurch gekennzeichnet, dass**
zumindest ein Kragen (8) der Gelenkstange (5) als ein am Ende der Gelenkstange eingebrachtes Langloch (9) ausgebildet ist, wobei sich die Öffnung des Langlochs (9) beim Überstreifen über den flachen Hinterkopf (15) des Gelenkbozens (7) reversibel weitet.

6. Protrusionsschiene nach einem der vorherigen Ansprüche,
**dadurch gekennzeichnet, dass**
die sich berührenden Flächen des Gelenkbolzens (7) und des Kragens (8) der Gelenkstange (5) als kantenfreie Gleitflächen geformt sind.

## Claims

1. Mandible protrusion rail for positioning the mandible (4) relative to the maxilla (1) of a patient having a maxilla partial rail (2) and a mandible partial rail (3), wherein joint rods (5) movably connect the two partial rails (2, 3) to each other, wherein the two partial rails (2, 3) have laterally attached joint pins (7) for holding the joint rods (5), wherein the joint pins (7) have a shoulder (10) facing the partial rail, a head (11) facing away from the partial rail and a neck (12) located between the shoulder (10) and head (11), wherein the joint pins each have a collar (8) on the ends thereof which, in the installed state of the joint rod (5), encloses the neck (12) of a joint pin (7), wherein the joint rod (5) is held between the shoulder (10) and the head (11), each having a larger cross-section than the neck (12), wherein there is a designed flexibility on the collar (8) of the joint rod (5) and/or on the head (11) of the joint pin (7), which, in the case of excess stressing by means of a laterally directed force exerted via the joint rod (5), first enables an evasive movement of the joint rod (5) **characterised in that** in the case of excess stressing, the designed flexibility makes non-destructive slipping of the collar (8) over the head (11) of the joint pin (7) possible.

2. Protrusion rail according to claim 1
**characterised in that**
the evasive movement of the joint rod (5) takes place in the direction of the head (11).

3. Protrusion rail according to claim 1 or 2 **characterised in that**
both partial rails (2, 3) with formed-on joint pins (7) are made of a harder plastic material, in particular of PMMA, than the joint rod (5), wherein the joint rod is, in particular, produced from a polyamide in an injection moulding process.

4. Protrusion rail according to any one of the preceding claims
**characterised in that**
the head (11) of a joint pin (7) is designed asymmetrically with a raised anterior head (14) and a flat posterior head (15), wherein the flat posterior head (15) faces the other partial rail.

5. Protrusion rail according to any one of the preceding claims
**characterised in that**
at least one collar (8) of the joint rod (5) is configured as an elongated hole (9) introduced at the end of the joint rod, wherein the opening of the elongated hole (9) on slipping over the flat posterior head (15) of the joint pain (7) reversibly widens.

6. Protrusion rail according to any one of the preceding claims
**characterised in that**
the surfaces of the joint pin (7) and the collar (8) of the joint rod (5) that are in contact with each other are formed as edge-free sliding surfaces.

## Revendications

1. Gouttière de protusion mandibulaire, destinée à positionner la mâchoire inférieure (4) par rapport à la mâchoire supérieure (1) d'un patient, comportant une gouttière partielle (2) de mâchoire supérieure et une gouttière partielle (3) de mâchoire inférieure, des tiges articulées (5) accouplant l'une à l'autre les deux gouttières partielles (2,3) de manière mobile, les deux gouttières partielles (2,3) comportant des axes d'articulation (7) montés latéralement, pour maintenir les tiges articulées (5), les axes d'articulation (7) comportant un épaulement (10) qui fait face à la gouttière partielle, une tête (11) qui est opposée à la gouttière partielle et un col (12) situé entre l'épaulement (10) et la tête (11), les tiges articulées comportant sur leurs extrémités chaque fois un collet (8), qui lorsque la tige articulée (5) est montée entoure le col (12) d'un axe d'articulation (7), la tige articulée (5) étant retenue entre l'épaulement (10) et la tête (11), qui ont chacun une section transversale supérieure à celle du col (12),
lors d'une exposition exagérée à une force dirigée latéralement, exercée par l'intermédiaire de la tige articulée (5), une souplesse structurellement prévue sur le collet (8) de la tige articulée (5) et/ou sur la tête (11) de l'axe d'articulation (7) permettant dans un premier temps un mouvement d'esquive de la tige articulée (5), **caractérisée en ce qu'en** cas de surcharge, la souplesse structurellement prévue permet alors un effleurement non destructif du collet (8) par-dessus la tête (11) de l'axe d'articulation (7).

2. Gouttière de protusion selon la revendication 1, **caractérisée en ce que**
le mouvement d'esquive de la tige articulée (5) s'effectue dans la direction de la tête (11).

3. Gouttière de protusion selon la revendication 1 ou 2, **caractérisée en ce que**
les deux gouttières partielles (2,3) avec des axes d'articulation (7) surmoulés sont fabriquées dans une matière plastique plus dure que la tige articulée (5), notamment en PMMA, la tige articulée étant produite en polyamide, notamment par procédé de moulage par injection.

4. Gouttière de protusion selon l'une quelconque des revendications précédentes,
**caractérisée en ce que**
la tête (11) d'un axe d'articulation (7) est conçue de forme asymétrique avec une avant-tête (14) en relief et une arrière-tête (15) plate, l'arrière-tête (15) plate faisant face à l'autre gouttière partielle.

5. Gouttière de protusion selon l'une quelconque des revendications précédentes,
**caractérisée en ce qu'**
au moins un collet (8) de la tige articulée (5) est conçu sous la forme d'un trou oblong (9) ménagé sur l'extrémité de la tige articulée, l'orifice du trou oblong (9) s'évasant de manière réversible lors de l'effleurement par-dessus l'arrière-tête (15) plate de l'axe d'articulation (7).

6. Gouttière de protusion selon l'une quelconque des revendications précédentes,
**caractérisée en ce que**
les surfaces en contact mutuel de l'axe d'articulation (7) et du collet (8) de la tige articulée (5) sont façonnées sous la forme de surfaces de coulissement sans arêtes.
